# EUROPEAN PATENT SPECIFICATION

(11) **EP 3 179 250 B1**
(45) Date of publication and mention of the grant of the patent: **12.02.2020**
(21) Application number: 15829574.1
(22) Date of filing: 06.08.2015
(51) Int. Cl.: G01N 33/50, G01N 27/62, G01N 33/493, G01N 33/53, G01N 33/543, G01N 33/68, G01N 33/88

(54) **METHOD AND KIT FOR TESTING FOR FOOD ALLERGY**
VERFAHREN UND KIT ZUR PRÜFUNG AUF LEBENSMITTELALLERGIE
PROCÉDÉ ET KIT DE TEST D'ALLERGIE ALIMENTAIRE

(30) Priority: 06.08.2014 JP 2014160891
(43) Date of publication of application: 14.06.2017
(73) Proprietor: The University of Tokyo, Tokyo 113-8654 (JP)
(72) Inventor: MURATA, Takahisa, Tokyo 113-8654 (JP); NAKAMURA, Tatsuro, Tokyo 113-8654 (JP); MAEDA, Shingo, Tokyo 113-8654 (JP)
(74) Representative: Griffin, Philippa Jane
(86) International application number: PCT/JP2015/072421
(87) International publication number: WO 2016/021704

(56) References cited:
- WO-A1-2010/104025
- JP-A- S6 111 664
- JP-A- 2010 002 416
- JP-A- 2013 530 185
- US-A1- 2010 209 962
- US-A1- 2010 209 962
- US-A1- 2011 311 990
- US-A1- 2012 021 437
- AWAD JOSEPH A ET AL: "Detection of the major urinary metabolite of prostaglandin D-2 in the circulation: Demonstration of elevated levels in patients with disorders of systemic mast cell activation", JOURNAL OF ALLERGY AND CLINICAL IMMUNOLOGY, ELSEVIER, AMSTERDAM, NL, vol. 93, no. 5, 1 January 1994 (1994-01-01), pages 817-824, XP002763938, ISSN: 0091-6749
- KIRSI M. JÄRVINEN ET AL: "Diagnostic oral food challenges: Procedures and biomarkers", JOURNAL OF IMMUNOLOGICAL METHODS., vol. 383, no. 1-2, 1 September 2012 (2012-09-01), pages 30-38, XP055434041, NL ISSN: 0022-1759, DOI: 10.1016/j.jim.2012.02.019
- YIZHONG ZHANG ET AL: "Simultaneous and high-throughput quantitation of urinary tetranor PGDM and tetranor PGEM by online SPE-LC-MS/MS as inflammatory biomarkers", JOURNAL OF MASS SPECTROMETRY., vol. 46, no. 7, 1 July 2011 (2011-07-01), pages 705-711, XP055433984, GB ISSN: 1076-5174, DOI: 10.1002/jms.1941
- TAKAHISA MURATA: 'Shokumotsu Allergy Chiryo Hoho to Byotai Marker no Tansaku' MISHIMA KAIUN KINEN ZAIDAN KENKYU HOKOKUSHO 2013, pages 33 - 35, XP008185549
- SONG WEN-LIANG ET AL: "Tetranor PGDM, an abundant urinary metabolite reflects biosynthesis of prostaglandin D2 in mice and humans.", THE JOURNAL OF BIOLOGICAL CHEMISTRY 11 JAN 2008, vol. 283, no. 2, 11 January 2008 (2008-01-11), pages 1179-1188, ISSN: 0021-9258

## Description

### Technical Field

The present invention relates to an *in vitro* examination method for simply and properly evaluating the risk of developing food allergy and seriousness of the symptoms thereof, as defined in the claims.

### Background Art

Food allergy refers to various allergic reactions caused by intake of allergens contained in food into bodies. As the symptoms of allergy, e.g., diarrhea, vomiting and dermatitis are mentioned. If the allergy becomes serious, a patient develops shock and results in death. The adult morbidity rate in this country is as high as 2.6%. The neonatal morbidity rate is further as high as 5.3% and severe cases are not low. Because of this, food allergy becomes a big issue.

As to how to develop food allergy, the following mechanism has been found.
i) T cells/B cells react with an allergen entered into a body and produce IgE, and
ii) When the allergen enters again in the body, mast cells combined with IgE secrete an active substance such as histamine (degranulation) and causes strong inflammation.

Egg, milk and wheat are well known allergens. Other than these, many foods can serve as an allergen. To prevent food allergy, there is no alternative but to identify the allergen and avoid eating it. The quality of life (QOL) of patients who cannot eat food in peace of mind is significantly impaired. In addition, elimination of responsible food will produce an adverse effect on nourishment, particularly in the case of children. Furthermore, due to a recent change in the living environment, the morbidity of food allergy increases and the symptoms thereof worsen. Elucidation of pathophysiological mechanism, development of therapeutic drugs of food allergy and establishment of experimental systems for evaluating food allergenicity call for urgent attention.

As one of the therapies for food allergy, a hyposensitization therapy is known. The hyposensitization therapy is a method of inducing immune tolerance by administering a diluted allergen to a patient under medical supervision. Many therapies for allergic diseases are symptomatic treatments; whereas, the hyposensitization therapy encourages the mechanism for developing allergic diseases and targets permanent cure. In this respect, the hyposensitization therapy have attracted attention. The hyposensitization therapy is started with a very low dose and the dose is gradually increased. At this time, in order to prevent occurrence of serious symptoms caused by allergen administered in excess of a threshold amount, a means for appropriately evaluating the seriousness of allergic symptoms in patients is required.

The diagnostic method for food allergy used at present is a method of measuring IgE in the blood. However, this method is good enough to estimate the presence or absence of an allergic reaction; however, a risk of developing food allergy and the degree of a symptom cannot be found. In other words, the blood level of IgE antibody does not agree with a risk of developing food allergy symptoms and seriousness thereof (Non-Patent Literature 1). This method further requires blood sampling. For blood sampling, patients must go to medical institutions and blood sampling itself is a great burden for infants.

### Citation List

Non-Patent Literature 1: Guidelines for medical examination of food allergy edited by the Health Labor Science Research group 2011 (http://www.allergy.go.jp/allergy/guideline/05/05 2011.pdf)

US 2012-021437 describes Tetranor-PGDM/PGJM Specific Immunogens, Antibodies, Tracers, Assay Kits and Methods for making the same; US 2010-209962 describes Tetranor PGDM: A Biomarker of PGD2 Synthesis *in vivo;* Awad et al., 1994, J. Allerg. Clin. Immunol., 93(5), pages 817-824, describes detection of the major urinary metabolite of prostaglandin D₂ in the circulation: Demonstration of elevated levels in patients with disorders of systemic mast cell activation; Jarvinen et al., 2012, J. Immunol. Methods, 282(1-2), pages 30-38, describes diagnostic oral food challenges: Procedures and biomarkers; Zhang et al., 2011, J. Mass. Spectrom., 46(7), pages 705-711 describes simultaneous and high-throughput quantitation of urinary tetranor PGDM and tetranor PGEM by online SPE-LC-MS/MS as inflammatory biomarkers.

### Summary of Invention

### Technical Problem

An object of the present invention is to provide an *in vitro* examination method for simply and properly evaluating the risk of developing food allergy, seriousness of the symptoms thereof and prognostic implication.

### Solution to Problem

In order to attain the aforementioned object, the present inventors focused on prostaglandin D₂ (PGD₂), which is a physiologically active substance most heavily produced by the mast cells. They studied about the possibility of evaluating a risk of developing food allergy and the seriousness thereof by measuring PGD₂. Unfortunately, it was difficult to detect PGD₂ since PGD₂ is metabolized in a brief (50 seconds), *in vivo.*

The present inventors conducted further studies. As a result, they found that a metabolite of PGD₂, namely, tetranor-PGDM, is excreted in urine and stably detected; and that the urinary concentration of PGD₂ has a correlation with the number of mast cells present in a living body, meaning that the urinary concentration of PGD₂ has a correlation with the risk of developing food allergy and seriousness thereof. They verified that if symptoms of food allergy are improved by administering a therapeutic drug, the urinary tetranor-PGDM amount decreases in accordance with the improvement. They further verified that since the tetranor-PGDM amount once increased by food allergy is maintained for a predetermined period even if allergic challenge is eliminated, examination can be made some time after the onset.

In addition, they found that tetranor-PGEM, which is a metabolite of prostaglandin E₂ (PGE₂), can be measured in a urine sample, and that, if the concentration change patterns of tetranor-PGDM and tetranor-PGEM are determined, food allergy can be distinguished from other inflammatory diseases. Based on the findings, the present invention was accomplished.

More specifically, the present invention relates to:
[1] An *in vitro* method for examining food allergy in a subject, including a step of measuring urinary tetranor-PGDM amount of the subject;
[2] The *in vitro* method according to [1], in which a higher tetranor-PGDM amount means that symptoms of food allergy are serious or have become more serious, or that the risk of developing food allergy is high or has become higher;
[3] The *in vitro* method according to [1] or [2], further including a step of measuring the urinary tetranor-PGEM amount of the subject;
[4] The *in vitro* method according to [3], in which a case where the tetranor-PGDM amount increases for a predetermined period and the tetranor-PGEM amount temporarily increases is determined as not other inflammatory diseases but food allergy;
[5] The *in vitro* method according to any one of [1] to [4], used in evaluation of a therapy or a therapeutic drug for food allergy, such as hyposensitization therapy;
[6] The *in vitro* method according to any one of [1] to [5], in which the tetranor-PGDM amount, and optionally the tetranor-PGEM amount, are measured by mass spectrometry or immunoassay;
[7] The *in vitro* method according to [6], including a pretreatment step of adding deuterated tetranor-PGDM, and optionally deuterated tetranor-PGEM, as an internal standard to urine of the subject; wherein the tetranor-PGDM amount, and optionally the tetranor-PGEM amount, are measured by mass spectrometry;
[8] The *in vitro* method according to any one of [1] to [7], in which activation of mast cells involved in food allergy is evaluated;
[9] Use of an anti-tetranor-PGDM antibody in the *in vitro* examination of a urine sample for markers of food allergy;
[10] Use according to [9], wherein the anti-tetranor-PGDM antibody is used with deuterated tetranor-PGDM;
[11] Use according to [9] or [10], wherein the anti-tetranor-PGDM antibody is used with: (i) an anti-tetranor-PGEM antibody; or (ii) an anti-tetranor-PGEM antibody and deuterated tetranor-PGEM;
[12] Use according to any one of [9] to [11], wherein the *in vitro* examination of a urine sample for markers of food allergy is for evaluating activation of mast cells involved in food allergy;
[13] Use according to any one of [9] to [12], wherein the anti-tetranor-PDGM antibody, and optionally the anti-tetranor-PGEM antibody, is contained in an examination stick;
[14] Use of urinary tetranor-PGDM, and optionally tetranor-PGEM, *in vitro* as a food allergy marker;
   and
[15] Use of urinary tetranor-PGDM, and optionally tetranor-PGEM, *in vitro* as a mast cell activation marker involved in food allergy.

### Advantageous Effects of Invention

According to the *in vitro* examination method of the present invention, not only the presence or absence of food allergy but also the risk of developing food allergy and the seriousness of symptoms can be appropriately evaluated by a method of simply measuring the tetranor-PGDM amount in a urine sample. According to this method, a risk is evaluated before symptoms are displayed and a prophylactic therapy can be made. In addition, since the responsiveness to a hyposensitization therapy is evaluated, allergen can be administered in as high a dose as possible within a safe range. In this way, a therapy can be efficiently applied.

The *in vitro* examination method according to the present invention requires no medical technology such as blood sampling. Thus, the examination method can be carried out at home by using an examination kit and stick as disclosed herein.

In the examination method according to the present invention, if the tetranor-PGEM amount in a urine sample is measured, food allergy can be distinguished from other inflammatory diseases.

### Brief Description of Drawings

[Figure 1] The figure shows the frequency of diarrhea and number of intestinal-tract mast cells of food allergy model mice prepared by antigen (OVA) challenge.
[Figure 2] The figure shows time-dependent change of urinary tetranor-PGDM and tetranor-PGEM concentration in food allergy model mice prepared by antigen (OVA) challenge.
[Figure 3] The figure shows time-dependent change of urinary tetranor-PGDM and tetranor-PGEM concentration after termination of antigen (OVA) challenge.
[Figure 4] The figure shows frequency of diarrhea and a urinary tetranor-PGDM concentration change in food allergy model mice administered with sodium cromoglycate.
[Figure 5] The figure shows frequency of diarrhea and a urinary tetranor-PGDM concentration change in food allergy model mice administered with dexamethasone.
[Figure 6] The figure shows the body weight change and the measurement results of Disease activity index in DSS-induced colitis model mice.
[Figure 7] The figure shows the measurement results of concentrations of urinary tetranor-PGDM and tetranor-PGEM in DSS-induced colitis model mice.
[Figure 8] The figure shows weight change and the length of the colon measured in TNBS-induced colitis model mice.
[Figure 9] The figure shows the measurement results of concentrations of urinary tetranor-PGDM and tetranor-PGEM in TNBS-induced colitis model mice.
[Figure 10] The figure shows images of the lung stained with H&E of an OVA-induced allergic bronchitis model mouse.
[Figure 11] The figure shows the measurement results of concentrations of urinary tetranor-PGDM and tetranor-PGEM in allergic bronchitis model mice.
[Figure 12] The figure shows the measurement results of ear thickness of DNFB-induced allergic contact dermatitis model mice.
[Figure 13] The figure shows the measurement results of concentrations of urinary tetranor-PGDM and tetranor-PGEM of allergic contact dermatitis model mice.
[Figure 14] The figure shows the mast cells engrafted in the colon and lung tissues of an NOG mouse to which human mast cells were transplanted.
[Figure 15] The figure shows the measurement results of concentrations of tetranor-PGDM and tetranor-PGEM excreted in the urine after mice having humanized mast cells are challenged with antigen/antibody.
[Figure 16] The figure shows the measurement results of concentrations of tetranor-PGDM and tetranor-PGEM excreted in the urine of mice, to which mouse mast cells (BMMC) or human mast cells (LAD2) were transplanted, after they were challenged with antigen/antibody.

### Description of Embodiments

### [Method for examining food allergy]

The *in vitro* method for examining food allergy according to the present invention includes a step of measuring urinary tetranor-PGDM amount of a subject. Tetranor-PGDM (9α-hydroxy-11,15-dioxo-13,14-dihydro-2,3,4,5-tetranor-prostan-1,20-dioic acid) is a metabolite of PGD₂. As is described in Examples, the urinary tetranor-PGDM amount increases with an increase of seriousness of food allergy symptoms, the number of mast cells in the tissue and activation thereof, and decreases with suppression of food allergy symptoms by administration of a therapeutic drug. Accordingly, if the urinary tetranor-PGDM amount is measured, not only the presence or absence of food allergy but also the seriousness thereof can be evaluated. In addition, the concentration of tetranor-PGDM in the urine of a person with food allergy is high even though antigen challenge is not performed. Accordingly, if the urinary tetranor-PGDM amount of a person who has not yet developed food allergy is measured, the risk of developing food allergy can be evaluated. US 2012/0021437 describes tetranor-PGDM/PGJM specific immunogens, antibodies, tracers, assay kits and methods for making the same; US 2010/0209962 describes tetranor PGDM as a biomarker of PGD2 synthesis *in vivo;* Awad, *et al.,* 1994, describes the detection of the major urinary metabolite of prostaglandin D₂ in the circulation and the demonstration of elevated levels in patients with disorders of systemic mast cell activation, J Allerg Clin Immunol, 93(5): 817-824; Jarvinen and Sicherer, 2012, describes diagnostic oral food challenges, procedures and biomarkers, J Immunol Methods, 383(1-2): 30-38; Zhang, et al., 2011, describes simultaneous and high-throughput quantitation of urinary tetranor PGDM and tetranor PGEM by online SPE-LC-MS/MS as inflammatory biomarkers, J Mass Spectrom, 46(7): 705-711.

In the specification, "food allergy" refers to various allergic reactions caused by taking an allergen contained in food into a body. As food allergy, allergies caused by e.g., egg, milk, crustaceans, wheat, fruits, nuts, fish and shellfish and buckwheat are well known. Allergies other than these are also included. Symptoms appear on e.g., the skin, mucosa, digestive organ and respiratory organ. Examples of typical symptoms include diarrhea, vomiting and dermatitis.

In the specification, the "subject" can be defined, for example, as a person who may possibly develop food allergy, a person who has already developed food allergy, a person who undergoes treatment for food allergy, a person who are taking a therapeutic drug for food allergy and a person who has not yet diagnosed as food allergy. The "subject" is not limited to a human and may be a mammal such as a mouse, a rat, a rabbit, a cat, a dog, a monkey, a pig, a sheep, a cow and a horse.

In an embodiment of the *in vitro* method for examining food allergy according to the present invention, a higher urinary tetranor-PGDM amount means that the symptoms are more serious or the risk of developing food allergy is higher. Whether the urinary tetranor-PGDM amount is higher or not may be determined by collecting urine a plurality of times from the same individual, measuring the urinary tetranor-PGDM amount and comparing the results. In this case, it is possible to evaluate a change in the seriousness of food allergy and risk of developing food allergy in the same individual. Whether the urinary tetranor-PGDM amount is higher or not may be evaluated by sampling urine from a plurality of persons, measuring the tetranor-PGDM amount of the urine samples and comparing the results. In this case, it is possible to evaluate whether the seriousness and risk of developing food allergy are higher than other persons.

In an embodiment of the *in vitro* method for examining food allergy according to the present invention, if the urinary tetranor-PGDM amount is higher than a predetermined value, it is determined that the symptoms are serious or the risk of developing food allergy is high. In contrast, if the urinary tetranor-PGDM amount is lower than the predetermined value, it is determined that the symptoms are mild or the risk of developing food allergy is low. The predetermined value (cutoff value) can be determined in accordance with a customary method by comparing the urinary tetranor-PGDM amount in urine sampled from a plurality of healthy persons or non-food allergy patients to the urinary tetranor-PGDM amount in urine sampled from a plurality of food-allergy patients.

In an embodiment, the *in vitro* method for examining food allergy according to the present invention is used in evaluation of therapeutic drugs to be administered to food-allergy patients and therapies to be applied to the patients. For example, a urine sample is collected at any time point before and after the initiation of a therapy and the tetranor-PGDM amount in the urine sample is measured. If the tetranor-PGDM amount decreases, the therapeutic drug or therapy is determined to be effective. In contrast, if the tetranor-PGDM amount does not decrease or increases, the therapeutic drug or therapy is determined to be ineffective. The decrease and increase in the tetranor-PGDM amount herein may not be a significant decrease or increase and may be sufficient if those skilled in the art determine that the amount has a tendency to decrease or increase.

In an embodiment, the *in vitro* method for examining food allergy according to the present invention may be used for evaluating the efficacy of therapeutic drugs for food allergy in animal experiments for drug development.

In an embodiment, the *in vitro* method for examining food allergy according to the present invention refers to a method of examining a sample collected from a subject in order to obtain information required for diagnosis. Such an examination method is carried out, for example, by laboratories.

The urine to be used for examination of the present invention can be collected in accordance with a customary method. Urine collected by a single sampling or pooled urine collected by two or more sampling may be used. The collected urine may be stored at room temperature or -40°C or less, for example, -80°C until examination time. The frozen urine can be rapidly thawed and subjected to examination.

Measurement of the urinary tetranor-PGDM amount can be carried out by any method as long as a predetermined substance in a solution can be detected or measured by the method. In the specification, the "measurement of the urinary tetranor-PGDM amount" includes not only accurate quantitative measurement but also detection of the presence or absence, and further includes just determining that the amount is larger or smaller than a predetermined amount. The urinary tetranor-PGDM amount may be corrected based on the amount of reference substance such as creatinine.

Examples of a method for measuring urinary tetranor-PGDM amount include, but are not limited to, immunoassay, agglutination, turbidimetry, western blotting, a surface plasmon resonance (SPR) method, a mass spectrometric method, a method using high performance liquid chromatography and a HPLC-MS/MS method. Of these, immunoassay for measuring the tetranor-PGDM amount based on the antigen-antibody reaction between an anti-tetranor-PGDM antibody and tetranor-PGDM in a urine sample is particularly simple and preferable.

In the immunoassay, an anti-tetranor-PGDM antibody labeled with a detectable marker or an antibody (secondary antibody) against the anti-tetranor-PGDM antibody labeled with a detectable marker, is used. The immunoassays are classified, depending upon the labelling method for an antibody, into enzyme immunoassay (EIA or ELISA), radioimmunoassay (RIA), fluorescent immunoassay (FIA), fluorescence polarization immunoassay (FPIA) and chemiluminescent immunoassay (CLIA). Any one of these assays can be used in the method of the present invention.

In ELISA, an antibody labeled with an enzyme such as peroxidase and alkaline phosphatase is used. In RIA, an antibody labeled with a radioactive material such as ¹²⁵I, ¹³¹I, ³⁵S, and ³H, is used. In FPIA, an antibody labeled with a fluorescent substance such as fluorescein isothiocyanate, rhodamine, dansyl chloride, phycoerythrin, tetramethylrhodamine isothiocyanate and a near infrared fluorescent material is used. In CLIA, an antibody labeled with a luminescent substance such as luciferase, luciferin and aequorin is used. Other than these, an antibody labeled with a nanoparticle such as gold colloid and quantum dot can be detected.

In the immunoassay, an anti-tetranor-PGDM antibody is labeled with biotin, and avidin or streptavidin labeled with e.g., an enzyme, is allowed to bind to biotin to detect the antibody.

Of the immunoassays, ELISA using an enzyme label is a method by which an antigen can be simply and quickly measured. ELISA includes a competitive method and a sandwich method. In the competitive method, an anti-tetranor-PGDM antibody is immobilized to a solid-phase carrier such as a microplate, and then, a urine sample and enzyme-labeled tetranor-PGDM are added to produce an antigen-antibody reaction. After washed once, the carrier is allowed to react with an enzyme substrate to produce a color and subjected to measurement of absorbance. If tetranor-PGDM amount in a urine sample is high, the intensity of the produced color is weak. In contrast, if the tetranor-PGDM amount in a urine sample is low, the intensity of the produced color is strong. Thus, the tetranor-PGDM amount can be obtained based on a calibration curve.

In the sandwich method, an anti-tetranor-PGDM antibody is immobilized to a solid-phase carrier, and then, a urine sample is added and reacted. Thereafter, another anti-tetranor-PGDM antibody labeled with an enzyme is added and reacted. After washing, an enzyme substrate is added, reacted to produce a color and subjected to measurement of absorbance. In this manner, the tetranor-PGDM amount can be obtained. In the sandwich method, the antibody immobilized to a solid phase carrier is reacted with tetranor-PGDM present in a urine sample. Thereafter, first, an unlabeled anti-tetranor-PGDM antibody (primary antibody) is added and then, an antibody (secondary antibody) against the unlabeled anti-tetranor-PGDM antibody labeled with an enzyme may further added.

As the enzyme substrate, if peroxidase is used as the enzyme, e.g., 3,3'-diaminobenzidine (DAB), 3,3'5,5'-tetramethylbenzidine (TMB) and o-phenylenediamine (OPD) can be used. In the case of alkaline phosphatase, e.g., p-nitropheny phosphate (NPP) can be used.

In the specification, the "solid-phase carrier" is not particularly limited as long as it is a carrier to which an antibody can be immobilized. Examples of the carrier include microtiter plates made of e.g., glass, a metal and a resin, a substrate, beads, a nitrocellulose membrane, a nylon membrane and a PVDF membrane. A target substance and an antibody can be immobilized to these solid phase carriers in accordance with a method known in the art.

An anti-tetranor-PGDM antibody, even if it is a monoclonal antibody or a polyclonal antibody, can be prepared in accordance with a method known in the art. The monoclonal antibody can be prepared, for example, by isolating an antibody-producing cell from a non-human mammal immunized with tetranor-PGDM and fused with e.g., a myeloma cell to produce a hybridoma, allowing the hybridoma to produce an antibody and purifying the antibody. The polyclonal antibody can be obtained from the serum of an animal immunized with tetranor-PGDM.

As the anti-tetranor-PGDM antibody, a ready-made antibody may be used.

In the *in vitro* method for examining food allergy according to the present invention, not only urinary tetranor-PGDM amount but also the tetranor-PGEM amount may be measured in a subject. Tetranor-PGEM (9,15-dioxo-11α-hydroxy-13,14-dihydro-2,3,4,5-tetranor-prostan-1,20-dioic acid) is a metabolite of PGE₂. As is described in Examples, in the case of food allergy, it was found that the tetranor-PGEM amount temporarily increases after onset and immediately decreases.

As is described in Examples, the behavior of the urinary tetranor-PGDM amount in other inflammatory diseases differs from that in food allergy. Accordingly, food allergy can be distinguished from other analogous inflammatory diseases (for example, enteritis, asthma, allergic dermatitis) by measuring the urinary tetranor-PGDM amount.

As is described in Examples, the behavior of the urinary tetranor-PGEM amount in other inflammatory diseases also differs from that in food allergy. Accordingly, analogous diseases can be further highly accurately distinguished by measuring both the urinary tetranor-PGDM amount and urinary tetranor-PGEM amount.

More specifically, a case where an increase of the urinary tetranor-PGDM amount is maintained for a predetermined period, and the urinary tetranor-PGEM amount temporarily increases and immediately decreases, the "subject" can be determined as food allergy. The predetermined period herein refers to, for example, 3 days or more, 5 days or more, 7 days or more, 10 days or more or 20 days or more after a causative substance of food allergy is taken. The "temporarily increases and immediately decreases" means that the amount temporarily increases and then decreases within 5 days, within 4 days or within 3 days.

In contrast, in a colitis model mouse prepared by oral administration of dextran sulfate sodium (DSS), the urinary tetranor-PGDM amount temporarily increases and immediately decreases; however, the tetranor-PGEM amount sharply increases and is thereafter continuously maintained at the high level. In the DSS enteritis model, inflammation mediated by neutrophils and macrophages is known to occur. In an ulcerous colitis model mouse induced by trinitrobenzene sulfonate (TNBS), the urinary tetranor-PGDM amount and urinary tetranor-PGEM amount both temporarily increase and immediately decrease. In an allergic bronchitis model mouse, the urinary tetranor-PGDM amount temporarily increases and immediately decreases; whereas, the urinary tetranor-PGEM amount does not change. Respiratory tract inflammation and bronchitis emerged in asthma are known to be a inflammation produced through eosinophils. In an allergic dermatitis model mouse, the urinary tetranor-PGDM amount and urinary tetranor-PGEM amount both increase and are maintained at the same level for a relatively long period.

The urinary tetranor-PGEM amount can be measured in the same manner as in the aforementioned method for measuring the urinary tetranor-PGDM amount.

In the *in vitro* method for examining food allergy according to the present invention, activation of mast cells involved in food allergy can be evaluated.

In the specification, the "activation of mast cells" refers to an increase in the number of mast cells and an increase of degranulation. As is described later in Examples, the urinary tetranor-PGDM amount increases as the number of mast cells increases and decreases during the period of suppressing degranulation. Also, the urinary tetranor-PGEM amount increases as the number of mast cells increases. Thus, activation of mast cells can be evaluated based on the urinary tetranor-PGDM amount and urinary tetranor-PGEM amount used as indicators.

For example, it is known that the symptoms of allergic rhinitis are developed in a mast cell-deficient mouse; however, food allergy is not virtually produced in the mast cell-deficient mouse. Likewise, it is known that food allergy is highly dependent on the mast cell, and that it is important to increase the number of mast cells in development and progression of food allergy. As a method of detecting activation of the mast cells characteristic to food allergy (particularly mast cells of the gastrointestinal mucosa), a method of measuring the histamine concentration in the blood is only available up to date. However, histamine in the blood disappears in 15 minutes. In this sense, a method for detecting activation of mast cells is virtually not present.

### [Examination kit for food allergy]

The examination kit for food allergy according to the present disclosure is a kit for examining food allergy by using the aforementioned examination method and contains an anti-tetranor-PGDM antibody.

The examination kit of the present disclosure contains reagents and devices required for measuring the tetranor-PGDM amount based on immunoassay using an anti-tetranor-PGDM antibody.

As an embodiment of the disclosure, the examination kit is used for measuring the tetranor-PGDM amount by the sandwich method and contains a microtiter plate; an anti-tetranor-PGDM antibody as a capture antibody; an anti-tetranor-PGDM antibody labeled with alkaline phosphatase or peroxidase; and an alkaline phosphatase substrate (e.g., NPP) or a peroxidase substrate (e.g., DAB, TMB, OPD).

The capture antibody and the labeled antibody recognize different epitopes.

In such a kit, first, the capture antibody is immobilized to the microtiter plate. To this, a urine sample is appropriately diluted, added and subjected to incubation and then the sample is removed and the plate is washed. Then, a labeled antibody is added and incubated. A substrate is added to produce color. Color is measured by e.g., a microtiter plate reader. In this manner, the tetranor-PGDM amount can be obtained.

In another embodiment of the disclosure, the examination kit is used for measuring the tetranor-PGDM amount by the sandwich method using a secondary antibody and contains a microtiter plate; an anti-tetranor-PGDM antibody as a capture antibody; anti-tetranor-PGDM antibody as a primary antibody; an antibody against the anti-tetranor-PGDM antibody labeled with alkaline phosphatase or peroxidase, as a secondary antibody; and an alkaline phosphatase substrate (e.g., NPP) or a peroxidase substrate (e.g., DAB, TMB, OPD).

The capture antibody and primary antibody recognize different epitopes.

In such a kit, first, the capture antibody is immobilized to the microtiter plate. To this, a urine sample is appropriately diluted, added and subjected to incubation and then the sample is removed and the plate is washed. Subsequently, the primary antibody is added and incubated. After washing, an enzyme labelled secondary antibody is further added and incubated. Then, a substrate is added to produce color. Color is measured by e.g., a microtiter plate reader. In this manner, the tetranor-PGDM amount can be obtained. Owing to the use of the secondary antibody, the reaction is amplified and detection sensitivity can be enhanced.

The examination kit may contain deuterated tetranor-PGDM used as an internal standard. The deuterated tetranor-PGDM is deuterium-labeled tetranor-PGDM. If this is used as an internal standard, when the urinary tetranor-PGDM amount is measured by a mass spectrometer, extraction efficiency and ionization efficiency per analysis can be corrected. Examples of the deuterated tetranor-PGDM include tetranor-PGDM-d6, tetranor-PGDM-d4 and tetranor-PGDM-d8.

Each examination kit may further contain e.g., a requisite buffer, an enzymatic reaction stop solution and a microplate reader.

The labeled antibody is not limited to an enzyme-labeled antibody and may be an antibody labeled with e.g., a radioactive material (e.g., ²⁵I, ¹³¹I, ³⁵S, ³H), a fluorescent substance (e.g., fluorescein isothiocyanate, rhodamine, dansyl chloride, phycoerythrin, tetramethylrhodamine isothiocyanate, a near infrared fluorescent material), a luminescent material (e.g., luciferase, luciferin, aequorin), or a nanoparticle (gold colloid, quantum dot). As the labeled antibody, a biotinylated antibody is used and a labeled avidin or streptavidin can be added to a kit.

The examination kit of the present disclosure may contain reagents and devices required for measuring the tetranor-PGEM amount in accordance with immunoassay using the anti-tetranor-PGEM antibody. The reagents and devices required for measuring the tetranor-PGEM amount are the same as used for measuring the tetranor-PGDM amount.

In this case, deuterated tetranor-PGEM used as an internal standard may be provided. The deuterated tetranor-PGEM is a deuterium labeled tetranor-PGEM. If this is used as an internal standard, when the urinary tetranor-PGEM amount is measured by a mass spectrometer, extraction efficiency and ionization efficiency per analysis can be corrected. Examples of the deuterated tetranor-PGDM include tetranor-PGEM-d6, tetranor-PGEM-d4 and tetranor-PGEM-d8.

The examination kit for food allergy according to the present disclosure can be used for evaluating activation of mast cells involved in food allergy.

### [Stick for use in examining urine sample]

The examination stick for food allergy according to the present disclosure is a stick-like examination drug for examining food allergy by use of the aforementioned examination method. The tetranor-PGDM amount in a urine sample is visualized by e.g., a colored line. As the examination stick, a stick known in the art can be used. More specifically, the examination stick, which is, for example, based on immunochromatography, may be constituted of an antibody container, which contains a first anti-tetranor-PGDM antibody labeled with e.g., gold colloid, and a determination section, in which a second anti-tetranor-PGDM antibody recognizing another epitope of tetranor-PGDM is immobilized linearly on e.g., cellulose membrane, the container and the section being connected with a narrow groove. If a urine sample is added to such a stick, the labeled antibody binds to tetranor-PGDM to form a tetranor-PGDM-labeled antibody complex in the antibody container. The complex moves to the determination section by way of the groove with the help of the capillary action. When the complex is captured by the second anti-tetranor-PGDM antibody immobilized, a red line emerges in the determination section due to the plasmon effect of gold colloid.

The determination section of the examination stick may have a line of a control to which an antibody against the labeled antibody is immobilized. Since the line of a control produces color regardless of the presence or absence of tetranor-PGDM, whether a test is correctly carried out or not can be checked.

The examination stick may further have e.g., an absorbent paper sheet for absorbing a urine sample and a drying agent.

The examination stick may have a labeled anti-tetranor-PGEM antibody in the antibody container and an anti-tetranor-PGEM antibody recognizing another epitope of tetranor-PGEM may be linearly immobilized in the determination section. If so, tetranor-PGDM and tetranor-PGEM can be measured at the same time.

According to such an examination stick, the urinary tetranor-PGDM amount and tetranor-PGEM amount can be easily measured at home and the risk of developing food allergy and the degree of food allergy can be evaluated.

The examination stick for food allergy according to the present disclosure can be used in evaluation of activation of mast cells involved in food allergy.

### [Food allergy marker and mast cell activation marker involved in food allergy]

In the specification, the "food allergy marker" refers to a substance used as an indicator of food allergy, and the risk of developing food allergy, seriousness of symptoms thereof and prognostic implication are shown by the amount of the substance. The "mast cell activation marker in food allergy" refers to a substance used as an indicator of (the presence or absence of) activation of mast cells involved in food allergy and the degree of activation of mast cells is shown by the amount of the substance.

In order to use urinary tetranor-PGDM or urinary tetranor-PGEM, *in vitro,* as the food allergy marker or the mast cell activation marker involved in food allergy, the urinary tetranor-PGDM amount or urinary tetranor-PGEM amount may be measured, as described in the *in vitro* method for examining food allergy according to the present invention. If the time-dependent change of the urinary tetranor-PGDM amount or urinary tetranor-PGEM amount is measured, the risk of developing food allergy, seriousness of symptoms of food allergy, prognostic implication and the presence or absence of activation of mast cells and the degree of activation can be efficiently estimated based on the time-dependent change pattern.

### Examples

Now, the present invention will be more specifically described based on Examples; however, the present invention is not particularly limited to these. Those skilled in the art can modify the present invention in various ways within the scope of the claims and such modifications are included in the scope of the present invention.

### 1. Materials and methods

### (1) Preparation of ovalbumin (ova)-induced food allergy model mouse

Model preparation: Wild type BALB/c mice (6-8 weeks old, female) were intraperitoneally sensitized with OVA by administering OVA (50 µg) twice at intervals of two weeks. Two weeks later, OVA (10 mg) was orally administered 10 times in total at intervals of two days. As a control, the mice sensitized with OVA was orally administered with physiological saline 10 times in total at intervals of two days.

Medication treatment: During the period of orally administering OVA to the mice sensitized with OVA in accordance with the aforementioned method, dexamethasone (2 mg/kg) or sodium cromoglycate (300 µg/mouse) were intraperitoneally administered every day.

Symptom evaluation: The number of mice which developed diarrhea within one hour after the OVA administration is determined in percentage.

Number of mast cells: The tissue of the intestinal tract was taken. Paraffin sections thereof were prepared and stained with chloro acetate esterase (CAE). Mast cells stained with red-pink color were counted under a microscope.

Urine sampling: 24 hours after oral administration of OVA or physiological saline, urine was collected by using a metabolism cage.

### (2) Preparation of dextran sulfate sodium (DSS)-induced colitis model mouse

Model preparation: 2% DSS was given to wild type C57BL/6 mice (6-8 weeks old, female) by allowing the mice to freely drink water for 7 days to develop colitis. Tap water was given to mice during the same period by allowing the mice to freely drink water and used as a control.

Symptoms evaluation: During the test period (10 days), body weight and Disease activity index (DAI) of the mice were measured every day. DAI was defined as follows. Point 0: normal stool, Point 1: loose stool, Point 2: very loose stool, Point 3: watery diarrhea, Point 4: hemorrhagic diarrhea
Urine sampling: Urine was collected for 24 hours by using a metabolism cage at intervals of two days.

### (3) Preparation of 2,4,6-trinitrobenzene sulfonate (TNBS)-induced colitis model mouse

Model preparation: Wild type C57BL/6 mice (6-8 weeks old, female) were intrarectally administered with 2.5% TNBS to induce colitis. As a control, mice were intracolically administered with a solvent (50% ethanol).

Symptom evaluation: After administration with TNBS the body weight of the mice was measured every day for 10 days.

Length of the colon: Day 3 and Day 10 after administration with TNBS, the mice were painlessly killed and length of the colons was measured.

Urine sampling: Urine was collected for 24 hours by using a metabolism cage at intervals of two days.

### (4) Preparation of OVA-induced allergic bronchitis model mouse

Model preparation: Wild type BALB/c mice (6-8 weeks old, female) were sensitized with OVA by intraperitoneally administering OVA (50 µg) twice at intervals of two weeks. Two weeks later, OVA (150 µg) was intranasally administered 10 times in total at intervals of two days. As a control, mice sensitized with OVA was intranasally administered with physiological saline 10 times in total at intervals of two days.

Symptom evaluation: 24 hours after the 10th OVA intranasal administration, the mice were painlessly killed and the lungs were excised out. Paraffin sections of the lung tissues were prepared, stained with H&E and then histopathologically evaluated.

Urine sampling: 24 hours after intranasal administration of of OVA or physiological saline, urine was collected by using a metabolism cage.

### (5) Preparation of allergic contact dermatitis model mouse

Model preparation: Wild type BALB/c mice (6-8 weeks old, female) were sensitized with an anti-DNP antibody by intradermally administering the anti-DNP antibody (1.25 µg) to the auricles. 24 hours later, 0.2% dinitrofluorobenzene (DNFB) was applied to the auricles to induce dermatitis.

Symptom evaluation: 30 minutes, 1 hour, 3 hours, 6 hours, 1-10 days after challenge with DNFB, the thickness of the auricles was measured. The difference from the thickness before challenge was determined as ear edema.

Urine sampling: Urine was collected for 24 hours by using a metabolism cage at intervals of two days.

### (6) Preparation of mast cell specific humanized mouse

Preparation of humanized mouse: Intestinal immunodeficiency mice, i.e., NOG mice (6-8 weeks old, female), were intravenously injected with human mast cell strain, i.e., LAD2 cells (5 × 10⁴ or 5 × 10⁵) to prepare mast cell specific humanized mice. As a control, mice were intravenously injected with mouse bone marrow-derived mast cells (BMMC). Evaluation of mast cells engraftment: Two weeks after the mast cell transplantation, the mice were painlessly killed. The numbers of mast cells in the colons and lungs of the mice stained with CAE were compared before and after the transplantation.

Urine sampling: Day 1, 3, 7, 14 and 21 after the transplantation, urine was collected for 24 hours by using a metabolism cage.

### (7) Pretreatment of urine

To mouse urine (100 µl) collected, 5 ng of tetranor-PGDM-d6 and tetranor-PGEM-d6 (deuterium-labeled tetranor-PGDM and tetranor-PGEM, respectively) were mixed as internal standards. The volume was controlled to be 1 ml with purified water and the pH was controlled to be 3 with hydrochloric acid. To a Sep-Pak Vac C18 cartridge equilibrated with ethanol and purified water, the urine prepared was poured. After washed with a 10% acetonitrile solution (6 ml) and hexane (6 ml), the urine was eluted with ethyl acetate (3 ml) and dried with nitrogen gas. The residue was dissolved in a 10% acetonitrile solution (100 µl) to prepare a measurement sample.

### (8) Measurement of urinary tetranor-PGDM and tetranor-PGEM

Using the urine sample pretreated, tetranor-PGDM and tetranor-PGEM were measured by a high performance liquid chromatography tandem mass spectrometer (HPLC-MS/MS). As the HPLC apparatus, Prominence system (a system controller CBM-20A, two feeding units LC-20AD, a column oven CTO-20A, an auto-sampler with cooling function SIL20AC, Shimadzu Corporation) was used. As the separation column, InertsilODS-3 (inner diameter 2.1 mm × length 150 mm (GL SCIENCES)) was used. The mobile phase was a concentration gradient of 0.02% acetic acid and acetonitrile. The flow rate was specified as 0.4 ml/min. The temperature of the column oven was set at 37°C and the temperature of the auto-sampler was set at 4°C. As the MS/MS section, a triple quadrupole mass spectrometer (LCMS-8030, Shimadzu Corporation) using electrospray ionization as an ion source. As a quantitative method, MRM (Multiple Reaction Monitoring) method was employed. Tetranor-PGDM (mass number 328) and tetranor-PGEM (mass number 328) were detected by using product ion m/z 309, which was produced by further decomposing the produced precursor ion m/z (mass number ÷ charge) 327 by collision induced dissociation (CID). Internal standards, tetranor-PGDM-d6 (mass number 334) and tetranor-PGEM-d6 (mass number 334), were detected by using product ion m/z 315, which was produced by further decomposing precursor ion m/z (mass number ÷ charge) 333 by CID. Since the chemical structures of tetranor-PGDM and tetranor-PGEM are extremely analogous, the precursor ions and the product ions are the same m/z values. Then, they were distinguished based on different retention times by HPLC. Data were analyzed by using software LabSolutions LCMS Ver. 5.53, attached to MS/MS. From the mass chromatogram prepared, the area of a peak derived from tetranor-PGDM or tetranor-PGEM was calculated. Quantification of each of the peaks was carried out based on the calibration curve prepared by a standard sample. At the time of the quantification, the extraction efficiency and ionization efficiency per analysis were corrected based on the area value of the peak derived from tetranor-PGDM-d6 or tetranor-PGEM-d6 introduced as an internal standard. The urinary tetranor-PGDM amount and tetranor-PGEM amount were corrected based on the urinary creatinine concentration and expressed by ng/mg Creatinine. The urinary creatinine concentration was measured by a measuring kit (LaboAssay Creatinine, Wako Pure Chemical Industries Ltd.).

### 2. Results

### (1) Correlation between degree of allergic symptoms and mast cells

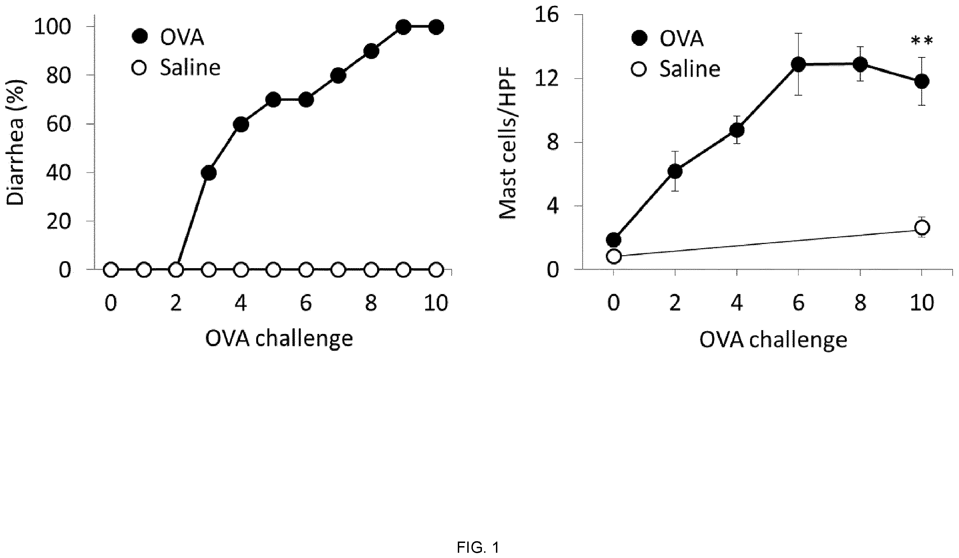

Figure 1 shows the frequency of diarrhea (left: n = 10) and number of mast cells (right: n = 4) in the colon tissue of food allergy model mice. As the number of times of antigen challenge increased, the frequency of diarrhea increased and the number of mast cells in the colon tissue increased.

### (2) Urinary tetranor-PGDM and tetranor-PGEM (1)

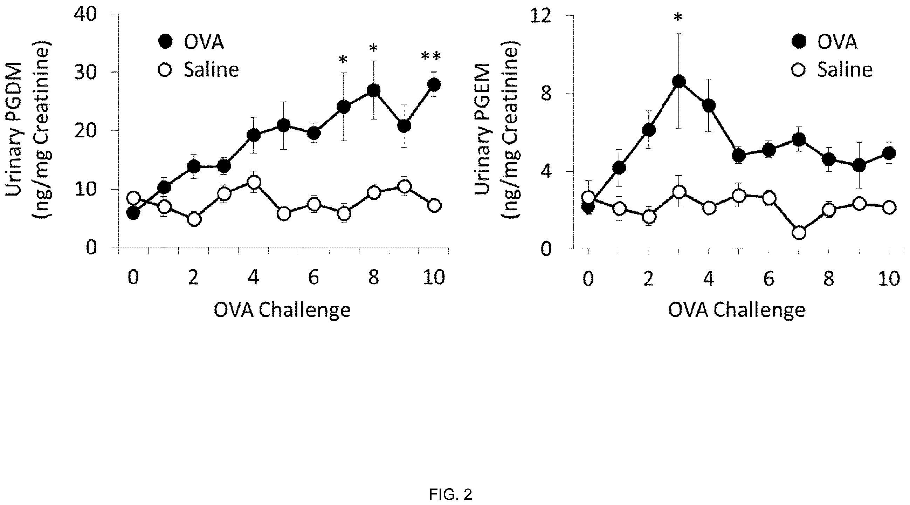

Figure 2 shows the time-dependent changes of urinary tetranor-PGDM and tetranor-PGEM in food allergy model mice. It is found that tetranor-PGDM increases as the number of times of antigen challenge increases (left: n = 10). In contrast, it was found that tetranor-PGEM temporarily increases after initiation of antigen challenge and immediately decreases (right: n = 10).

### (3) Urinary tetranor-PGDM and tetranor-PGEM (2)

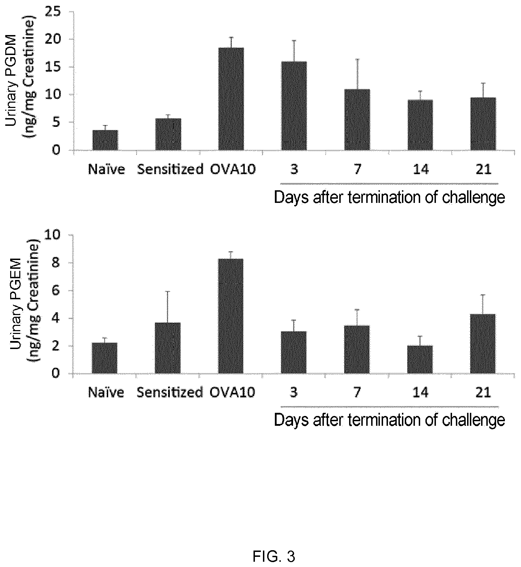

Figure 3 shows the time-dependent changes of urinary tetranor-PGDM and tetranor-PGEM for a predetermined period after termination of antigen challenge. The urinary PGDM concentration was on a downward trend; however, within three weeks after termination of challenge, the PGDM concentration in excreted urine was significantly high (upper graph: n = 7). In contrast, it was found that PGEM decreases immediately after the termination of challenge (lower graph: n = 7).

### (4) Specificity of urinary tetranor-PGDM to mast cells

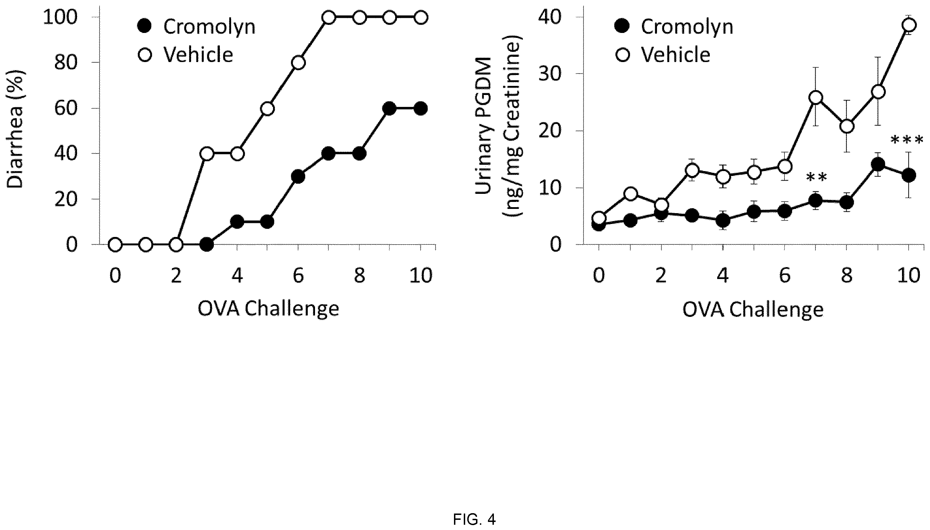

Figure 4 shows a change of urinary tetranor-PGDM when sodium cromoglycate (cromolyn) was intraperitoneally administered at a dose of 300 µg per day per mice during the OVA oral administration period. Cromolyn stabilizes the cell membrane of mast cells and thereby suppresses degranulation. Administration of cromolyn suppressed food allergy symptoms induced by OVA (left: n = 5) and significantly decreased the tetranor-PGDM amount excreted in the urine (right: n = 5).

### (5) Responsiveness of urinary tetranor-PGDM to therapy

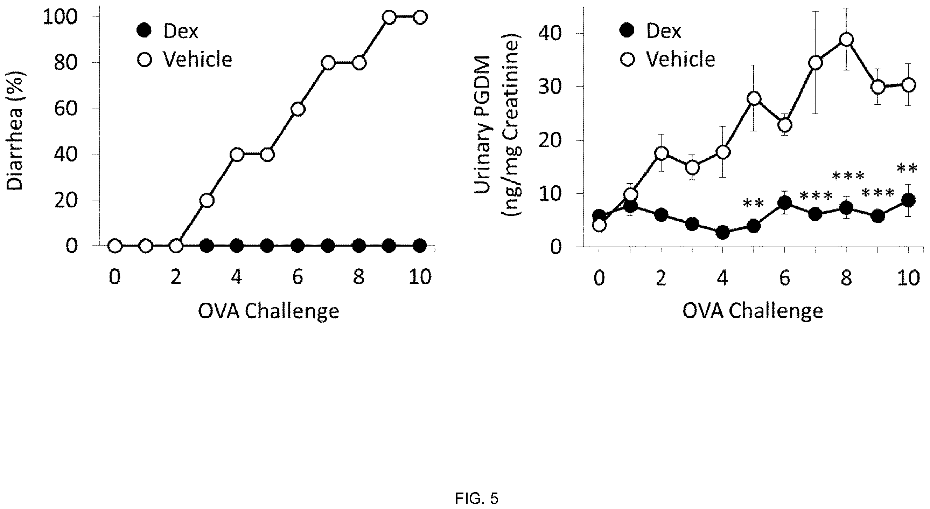

Figure 5 shows a change of urinary tetranor-PGDM when steroidal anti-inflammatory drug, dexamethasone (DEX), for use in therapy for allergic diseases was intraperitoneally administered at a dose of 2 mg per day per body weight (1 kg). Administration of DEX suppressed food allergy symptoms induced by OVA (left: n = 5); at the same time, significantly decreased the tetranor-PGDM amount excreted in the urine (right: n = 5).

### (6) Differentiation of analogous diseases based on urinary tetranor-PGDM and tetranor-PGEM concentrations (1)

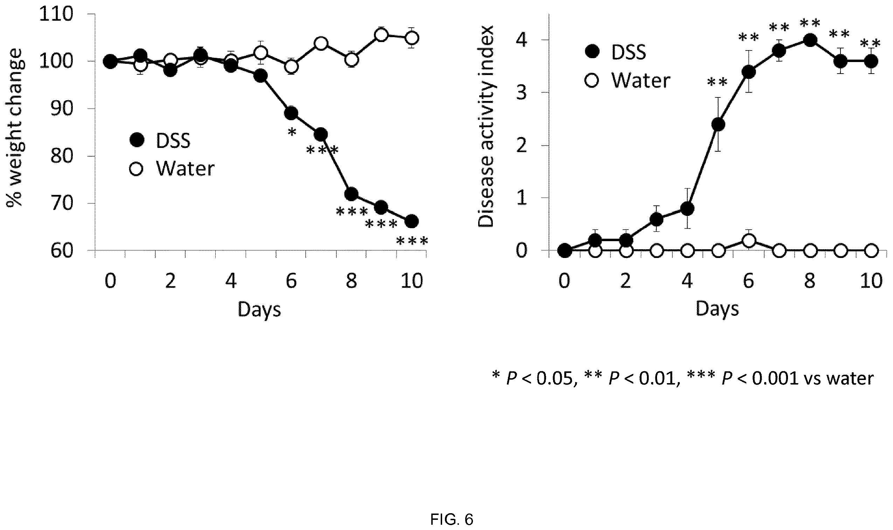

Figure 6 shows a change in body weight (left: n = 5) and a change in seriousness (right: n = 5) of a DSS-induced colitis model. It was observed that weight is reduced and colitis become exacerbated by administration of DSS.

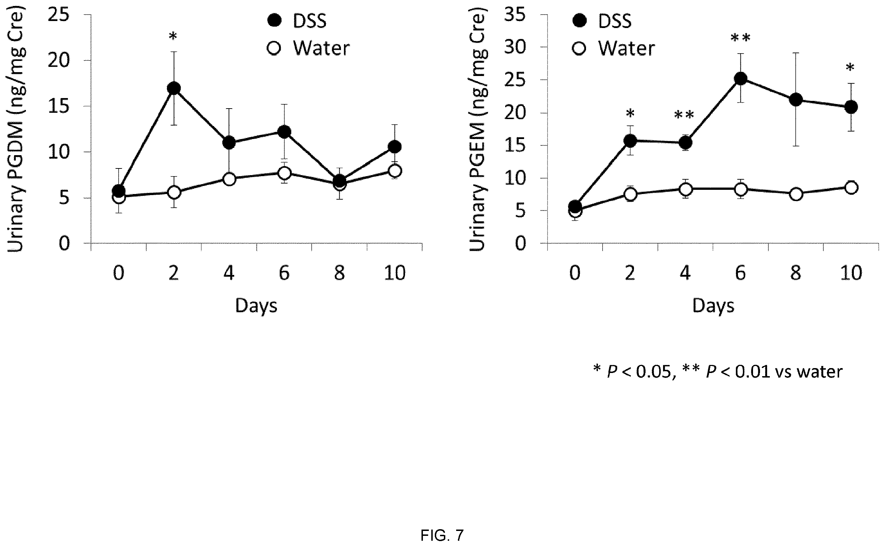

Figure 7 shows the time-dependent changes of urinary tetranor-PGDM and tetranor-PGEM in a DSS-induced colitis model. The urinary tetranor-PGDM concentration temporarily increased after DSS administration and immediately decreased (left: n = 5). In contrast, the tetranor-PGEM concentration sharply increased after initiation of DSS administration and the increased level was maintained (right: n = 5).

### (7) Differentiation of analogous diseases based on urinary tetranor-PGDM and tetranor-PGEM concentrations (2)

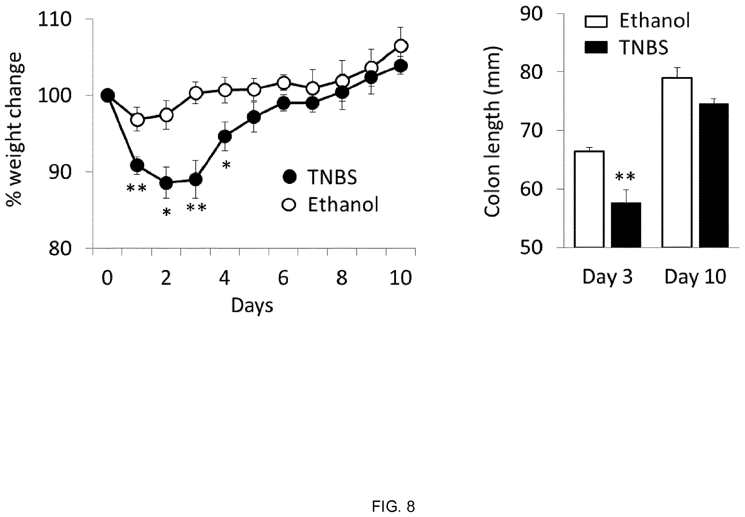

Figure 8 shows the weight change (left: n = 5) and the measurement results of colon length (right: n = 5) of TNBS-induced colitis model mice. It was observed that weight loss and shrinkage of the colon caused by TNBS.

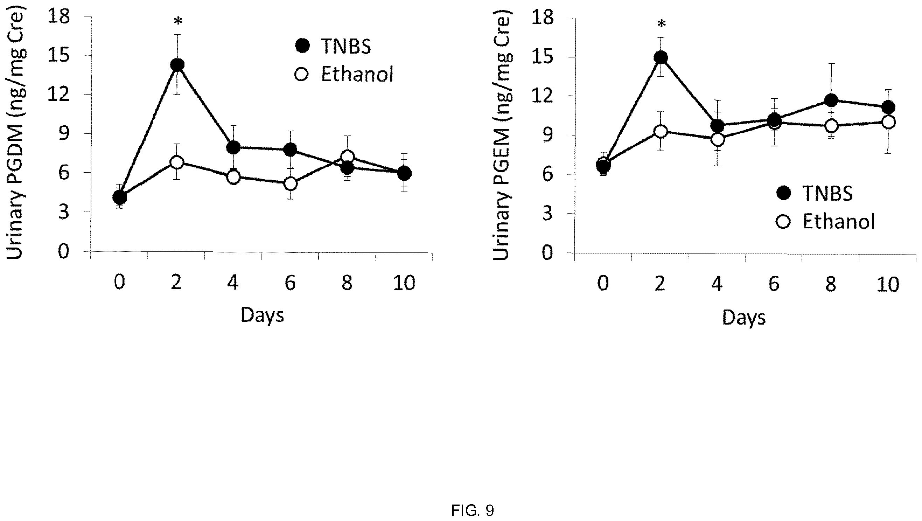

Figure 9 shows the time-dependent change of urinary tetranor-PGDM and tetranor-PGEM in TNBS-induced colitis model mice. A transient increase in concentration was observed in both tetranor-PGDM (left: n = 5) and tetranor-PGEM (right: n = 5).

### (8) Differentiation of analogous diseases based on urinary tetranor-PGDM and tetranor-PGEM concentrations (3)

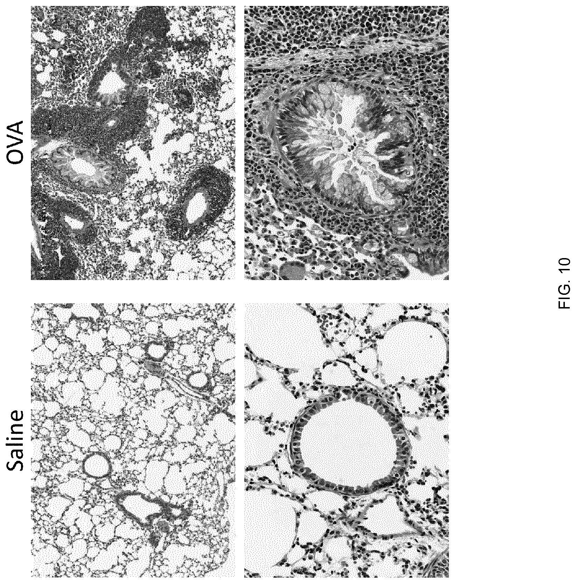

Figure 10 shows images of the lung stained with H&E of an OVA-induced allergic bronchitis model mouse. Infiltration of inflammatory cells and hyperplasia of bronchial goblet cells by OVA administration were observed. (right).

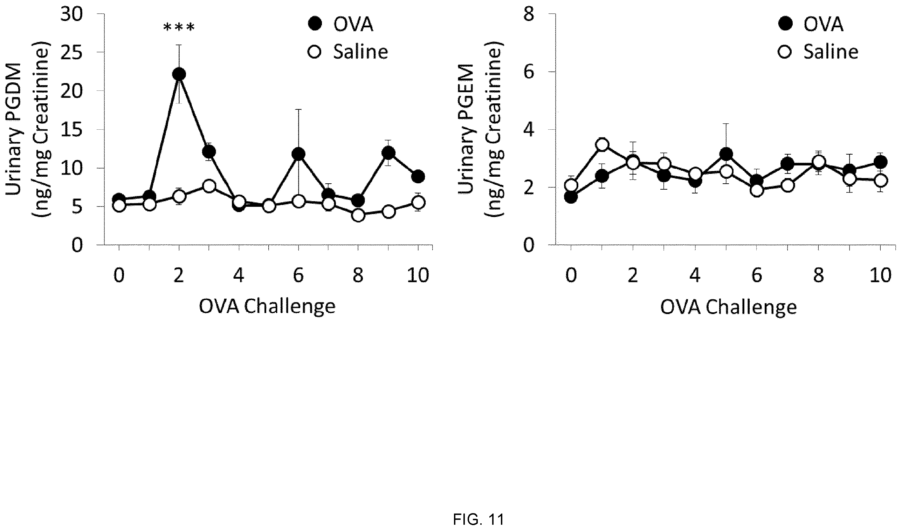

Figure 11 shows the time-dependent change of urinary tetranor-PGDM and tetranor-PGEM in an allergic bronchial inflammation model. The concentration of urinary tetranor-PGDM temporarily increased (left: n = 5). In contrast, the concentration of tetranor-PGEM (right: n = 5) was nearly unchanged.

### (9) Differentiation of analogous diseases based on urinary tetranor-PGDM and tetranor-PGEM concentrations (4)

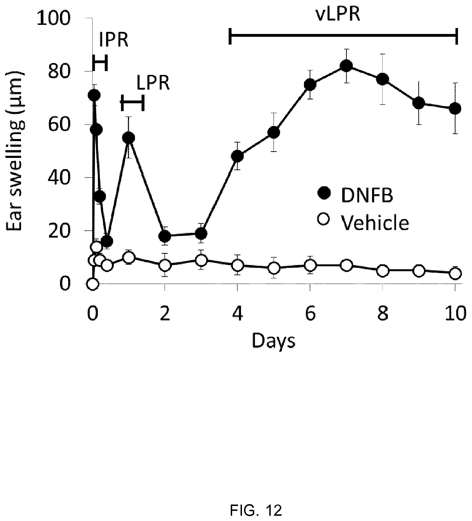

Figure 12 shows swelling of ear produced in a DNFB-induced allergic contact dermatitis model. When DNFB was applied, swelling of the ear emerged in three phases: immediate phase reaction (IPR), late phase reaction (LPR), very-late phase reaction (vLPR) (n = 5).

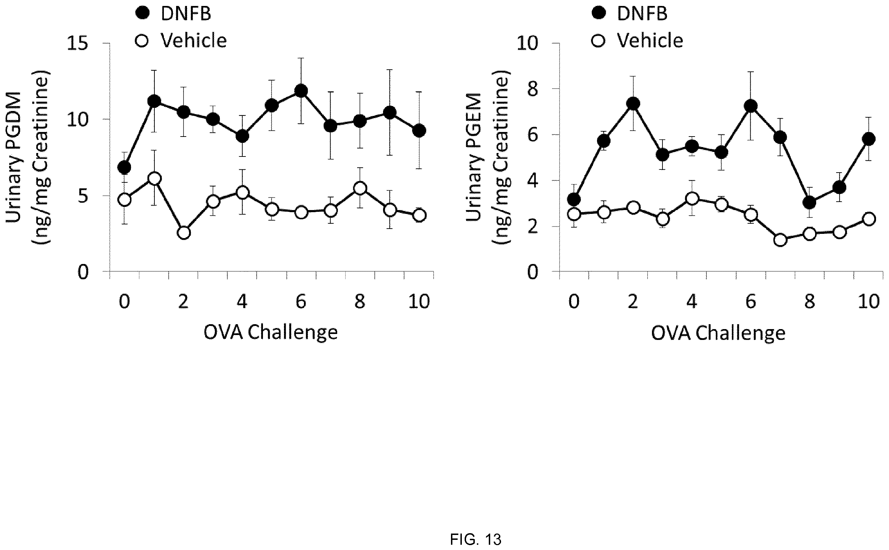

Figure 13 shows the time-dependent change of urinary tetranor-PGDM and tetranor-PGEM in an allergic dermatitis model. Slight increases in urinary tetranor-PGDM (right: n = 5) and urinary tetranor-PGEM (left: n = 5) were observed and the increased levels were maintained for a long period.

### (10) Urinary tetranor-PGDM and tetranor-PGEM in mast cell specific humanized mouse

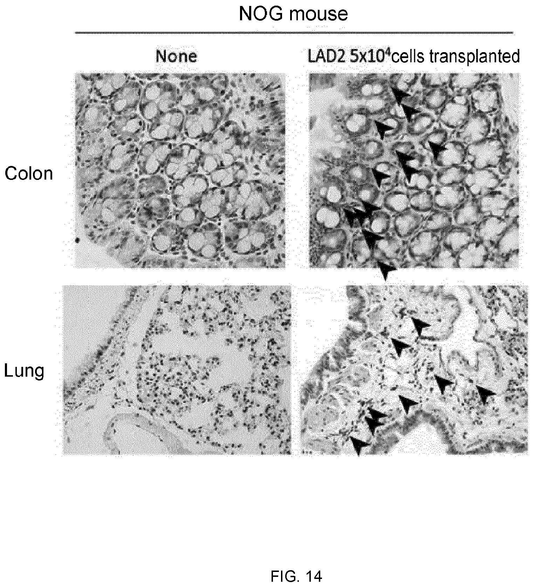

Figure 14 shows human mast cells (LAD2) engrafted in the colon and lung tissue of a hyper immunodeficiency (NOG) mouse (Figure 7, left: n = 3).

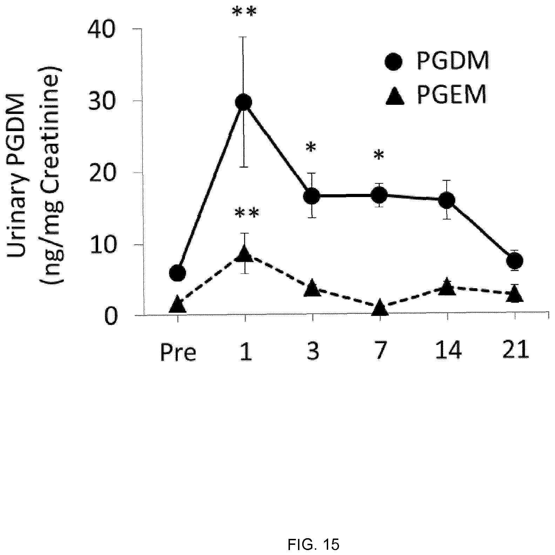

Figure 15 shows the time-dependent change of tetranor-PGDM and tetranor-PGEM excreted in urine after antigen/antibody challenge to mast cell humanized mice. The urinary tetranor-PGDM concentration immediately increased after the challenge and the high level was maintained for two weeks (●: n = 4). In contrast, the tetranor-PGEM concentration temporarily increased after the challenge and immediately (6 hours later) decreased (▲: n = 4).

From these results, it was found that urinary tetranor-PGDM and tetranor-PGEM are useful also as a human allergic marker.

### (11) Production of urinary tetranor-PGDM/PGEM by human or mouse mast cells

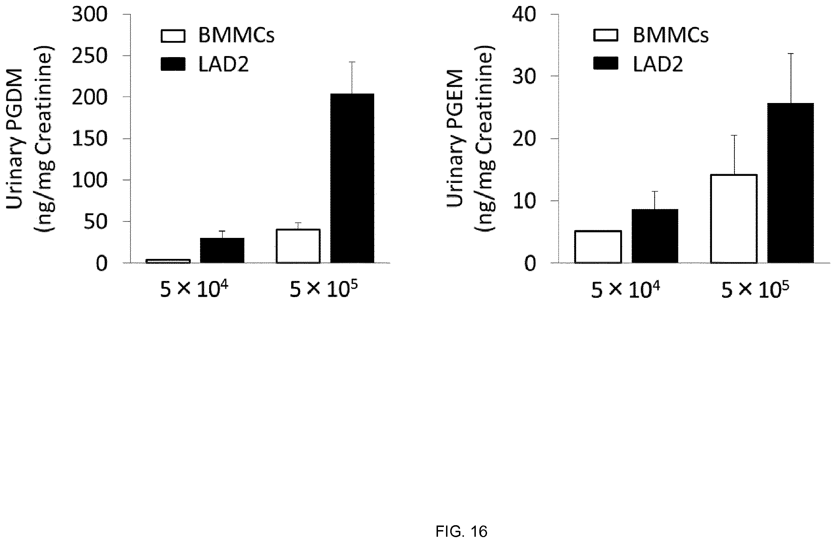

To NOG mice, mouse mast cells (BMMC) and human mast cells (LAD2) were separately transplanted. After challenge by antigen/antibody, urinary tetranor-PGDM and tetranor-PGEM were excreted as shown in Figure 16. In the mouse transplanted with the human mast cells, tetranor-PGDM was excreted in an amount about 10 times as large as the mouse transplanted with the mouse mast cells (left: n = 5); the concentration of tetranor-PGEM excreted was about 2.5 time as large as the mouse transplanted with the mouse mast cells (right: n = 5).

Also from the results, it was found that urinary tetranor-PGDM and tetranor-PGEM are useful as a human allergic marker.

## Claims

1. An *in vitro* method for examining food allergy in a subject, comprising a step of measuring the amount of tetranor-PGDM in a urine sample obtained from the subject.

2. The *in vitro* method according to Claim 1, wherein a higher tetranor-PGDM amount means that symptoms of food allergy are serious or have become more serious, or that the risk of developing food allergy is high or has become higher.

3. The *in vitro* method according to Claim 1 or 2, further comprising a step of measuring the amount of tetranor-PGEM in a urine sample obtained from the subject.

4. The *in vitro* method according to Claim 3, wherein an increase in the amount of tetranor-PGDM over a predetermined period and a temporary increase in the amount of tetranor-PGEM over a predetermined period indicates that the subject has or is at risk of having a food allergy and not another inflammatory disease.

5. The *in vitro* method according to any one of Claims 1 to 4, for evaluating the effectiveness of a therapy or a therapeutic drug for food allergy, such as a hyposensitization therapy.

6. The *in vitro* method according to any one of Claims 1 to 5, wherein the tetranor-PGDM amount, and optionally the tetranor-PGEM amount, are measured by mass spectrometry or immunoassay.

7. The *in vitro* method according to Claim 6, comprising a pretreatment step of adding deuterated tetranor-PGDM, and optionally deuterated tetranor-PGEM, as an internal standard to the urine sample of the subject; wherein the tetranor-PGDM amount, and optionally the tetranor-PGEM amount, are measured by mass spectrometry.

8. The *in vitro* method according to any one of Claims 1 to 7, for evaluating activation of mast cells involved in food allergy.

9. Use of an anti-tetranor-PGDM antibody in the *in vitro* examination of a urine sample for markers of food allergy.

10. Use according to Claim 9, wherein the anti-tetranor-PGDM antibody is used with deuterated tetranor-PGDM.

11. Use according to Claim 9 or Claim 10, wherein the anti-tetranor-PGDM antibody is used with: (i) an anti-tetranor-PGEM antibody; or (ii) an anti-tetranor-PGEM antibody and deuterated tetranor-PGEM.

12. Use according to any one of Claims 9 to 11, wherein the *in vitro* examination of a urine sample for markers of food allergy is for evaluating activation of mast cells involved in food allergy.

13. Use according to any one of Claims 9 to 12, wherein the anti-tetranor-PDGM antibody, and optionally the anti-tetranor-PGEM antibody, is contained in an examination stick.

14. Use of urinary tetranor-PGDM, and optionally tetranor-PGEM, *in vitro* as a food allergy marker.

15. Use of urinary tetranor-PGDM, and optionally tetranor-PGEM, *in vitro* as a marker of mast cell activation involved in food allergy.

## Patentansprüche

1. *In-vitro*-Verfahren zur Untersuchung von Lebensmittelallergie bei einem Subjekt, umfassend einen Schritt des Messens der Menge an Tetranor-PGDM in einer von dem Subjekt erhaltenen Urinprobe.

2. *In-vitro*-Verfahren gemäß Anspruch 1, wobei eine größere Menge an Tetranor-PGDM bedeutet, dass Symptome von Lebensmittelallergie ernst sind oder ernster geworden sind oder dass das Risiko der Entwicklung von Lebensmittelallergie hoch ist oder höher geworden ist.

3. *In-vitro*-Verfahren gemäß Anspruch 1 oder 2, ferner umfassend einen Schritt des Messens der Menge an Tetranor-PGEM in einer von dem Subjekt erhaltenen Urinprobe.

4. *In-vitro*-Verfahren gemäß Anspruch 3, wobei eine Zunahme der Menge an Tetranor-PGDM über einen vorbestimmten Zeitraum und eine vorübergehende Zunahme der Menge an Tetranor-PGEM über einen vorbestimmten Zeitraum anzeigt, dass das Subjekt ein Risiko aufweist oder darunter steht, eine Lebensmittelallergie und nicht eine andere entzündliche Erkrankung aufzuweisen.

5. *In-vitro*-Verfahren gemäß einem der Ansprüche 1 bis 4 zur Bestimmung der Wirksamkeit einer Therapie oder eines therapeutischen Arzneimittels für Lebensmittelallergie, wie z. B. einer Hyposensibilisierungstherapie.

6. *In-vitro*-Verfahren gemäß einem der Ansprüche 1 bis 5, wobei die Menge an Tetranor-PGDM und gegebenenfalls die Menge an Tetranor-PGEM durch Massenspektrometrie oder Immunassay gemessen werden.

7. *In-vitro*-Verfahren gemäß Anspruch 6, umfassend einen Vorbehandlungsschritt des Zugebens von deuteriertem Tetranor-PGDM und gegebenenfalls deuteriertem Tetranor-PGEM als interner Standard zu der Urinprobe des Subjekts; wobei die Menge an Tetranor-PGDM und gegebenenfalls die Menge an Tetranor-PGEM durch Massenspektrometrie gemessen werden.

8. *In-vitro*-Verfahren gemäß einem der Ansprüche 1 bis 7 zur Bestimmung der Aktivierung von bei Lebensmittelallergie beteiligten Mastzellen.

9. Verwendung eines Anti-Tetranor-PGDM-Antikörpers bei der *In-vitro*-Untersuchung einer Urinprobe auf Marker von Lebensmittelallergie.

10. Verwendung gemäß Anspruch 9, wobei der Anti-Tetranor-PGDM-Antikörper mit deuteriertem Tetranor-PGDM verwendet wird.

11. Verwendung gemäß Anspruch 9 oder Anspruch 10, wobei der Anti-Tetranor-PGDM-Antikörper mit:
(i) einem Anti-Tetranor-PGEM-Antikörper; oder
(ii) einem Anti-Tetranor-PGEM-Antikörper und deuteriertem Tetranor-PGEM verwendet wird.

12. *In-vitro*-Verfahren gemäß einem der Ansprüche 9 bis 11, wobei die *In-vitro*-Untersuchung einer Urinprobe auf Marker von Lebensmittelallergie zur Bestimmung der Aktivierung von bei Lebensmittelallergie beteiligten Mastzellen ist.

13. *In-vitro*-Verfahren gemäß einem der Ansprüche 9 bis 12, wobei der Anti-Tetranor-PGDM-Antikörper und gegebenenfalls der Anti-Tetranor-PGEM-Antikörper in einem Untersuchungsstift enthalten sind.

14. Verwendung von Harn-Tetranor-PGDM und gegebenenfalls Tetranor-PGEM *in vitro* als Lebensmittelallergie-Marker.

15. Verwendung von Harn-Tetranor-PGDM und gegebenenfalls Tetranor-PGEM *in vitro* als Marker von bei Lebensmittelallergie beteiligter Mastzellenaktivierung.

## Revendications

1. Procédé *in vitro* pour l'analyse d'une allergie alimentaire chez un sujet, comprenant une étape consistant à mesurer la quantité de tétranor-PGDM dans un échantillon d'urine obtenu à partir du sujet.

2. Procédé *in vitro* selon la revendication 1, dans lequel une quantité plus élevée de tétranor-PGDM signifie que les symptômes d'allergie alimentaire sont graves ou sont devenus plus graves ou que le risque de développement d'allergie alimentaire est élevé ou est devenu plus élevé.

3. Procédé *in vitro* selon la revendication 1 ou 2, comprenant en outre une étape consistant à mesurer la quantité de tétranor-PGEM dans un échantillon d'urine obtenu à partir du sujet.

4. Procédé *in vitro* selon la revendication 3, dans lequel une augmentation de la quantité de tétranor-PGDM sur une période prédéfinie et une augmentation temporaire de la quantité de tétranor-PGEM sur une période prédéfinie indiquent que le sujet a ou présente un risque d'avoir une allergie alimentaire et pas une autre maladie inflammatoire.

5. Procédé *in vitro* selon l'une quelconque des revendications 1 à 4, pour l'évaluation de l'efficacité d'une thérapie ou d'un médicament thérapeutique contre une allergie alimentaire, tels qu'une thérapie par hyposensibilisation.

6. Procédé *in vitro* selon l'une quelconque des revendications 1 à 5, dans lequel la quantité de tétranor-PGDM, et éventuellement la quantité de tétranor-PGEM, sont mesurées par spectrométrie de masse ou immunoessai.

7. Procédé *in vitro* selon la revendication 6, comprenant une étape de prétraitement consistant à ajouter du tétranor-PGDM deutéré, et éventuellement du tétranor-PGEM deutéré, en tant qu'étalon interne à l'échantillon d'urine du sujet ; dans lequel la quantité de tétranor-PGDM, et éventuellement la quantité de tétranor-PGEM, sont mesurées par spectrométrie de masse.

8. Procédé *in vitro* selon l'une quelconque des revendications 1 à 7, pour l'évaluation de l'activation de mastocytes impliquée dans une allergie alimentaire.

9. Utilisation d'un anticorps anti-tétranor-PGDM dans l'analyse *in vitro* d'un échantillon d'urine en ce qui concerne des marqueurs d'allergie alimentaire.

10. Utilisation selon la revendication 9, dans laquelle l'anticorps anti-tétranor-PGDM est utilisé avec du tétranor-PGDM deutéré.

11. Utilisation selon la revendication 9 ou la revendication 10, dans laquelle l'anticorps anti-tétranor-PGDM est utilisé avec : (i) un anticorps anti-tétranor-PGEM ; ou (ii) un anticorps anti-tétranor-PGEM et du tétranor-PGEM deutéré.

12. Utilisation selon l'une quelconque des revendications 9 à 11, dans laquelle l'analyse *in vitro* d'un échantillon d'urine en ce qui concerne des marqueurs d'allergie alimentaire sert à l'évaluation de l'activation de mastocytes impliquée dans une allergie alimentaire.

13. Utilisation selon l'une quelconque des revendications 9 à 12, dans laquelle l'anticorps anti-tétranor-PGDM, et éventuellement l'anticorps anti-tétranor-PGEM, sont contenus dans une bandelette d'analyse.

14. Utilisation de tétranor-PGDM, et éventuellement de tétranor-PGEM, urinaires *in vitro* en tant que marqueurs d'allergie alimentaire.

15. Utilisation de tétranor-PGDM, et éventuellement de tétranor-PGEM, urinaires *in vitro* en tant que marqueurs d'activation de mastocytes impliquée dans une allergie alimentaire.
